# EUROPEAN PATENT APPLICATION

(11) **EP 4 046 681 A1**
(43) Date of publication of application: **24.08.2022**
(21) Application number: 21158737.3
(22) Date of filing: 23.02.2021
(51) Int. Cl.: A61N 1/375

(54) **INTRA-CARDIAC DEVICE**

(71) Applicant: BIOTRONIK SE & Co. KG, 12359 Berlin (DE)
(72) Inventor: MIDGETT, Madeline Anne, Portland, OR 97213 (US); WHITTINGTON, R. Hollis, Portland, OR 97202 (US); MUESSIG, Dirk, West Linn, Oregon 97068 (US); AUSTIN, Eric, Portland, OR 97221 (US); TAFF, Brian M., Portland, OR 97217 (US); HUGHES, Devan, Tualatin, Oregon 97026 (US)
(74) Representative: Biotronik Corporate Services SE

(57) **Abstract**

A novel intracardiac device for implantation within the appendage (10) of an atrium of a patient's heart is described comprising a housing (20) and an electrode (24) projecting from the distal end of the housing (20), wherein a fixation structure is located at the proximal region of the housing (20) and extending therefrom, wherein the fixation structure comprises
- at least two flexible arms (30, 40, 60, 70), wherein the proximal end (33, 43, 63, 73) of each flexible arm is adapted in its deployed state such that it is in contact and supported by the atrial appendage wall, wherein each flexible arm is preloaded such that the intracardiac device and its electrode (24) is pressed against the atrial appendage wall in the region of a distal tip (12) of the appendage, and/or
- at least one coil spring arm (50), wherein each coil spring arm is adapted in its deployed state such that at least two sections (53, 54, 55, 56, 57) of each coil spring arm are in contact and supported by the atrial appendage wall, wherein the coil spring arm is preloaded such that the intracardiac device and its electrode (24) is pressed against the atrial appendage wall in the region of a distal tip (12) of the appendage.

## Description

The present invention refers to an implantable intracardiac device, such as an implantable intracardiac pacemaker.

Active or passive intracardiac medical devices (IMD), for example implantable intracardiac pacemakers (also known as leadless pacemakers), are well known miniaturized medical devices which are entirely implanted into a heart's chamber or atrium of a patient. Intracardiac pacemakers are used for patients who suffer from a bradycardia. Intracardiac devices apply electrical stimulation in the form of pulses to the heart in order to generate a physiologically appropriate heartrate (intracardiac pacemakers) and/or in the form of shocks for cardioversion or defibrillation in order to restore a more normal heart rhythm. Alternative or additional functions of intracardiac devices comprise providing other electrical or electromagnetic signals to the heart or its surrounding tissue, sensing electrical or electromagnetic signals or other physiological parameters of the heart and/or its surrounding tissue.

Intracardiac device fixation mechanisms in the field are currently only used for ventricular implantation. As intracardiac pacemakers use is expanded to dual-chamber applications, specialized atrial fixation methods are also needed. Atrial anatomy dictates safe and reliable fixation approaches. The right atrium lateral wall and appendage is extremely thin and sparsely covered with pectinate muscle. In contrast, the septal and posterior right atrium walls are smooth without any pectinate muscles.

Document US 8,700,181 discloses an intracardiac medical device for implantation into one atrium comprising a stabilizing intracardiac device extension connected to the housing. The stabilizing extension includes a stabilizer arm and/or an appendage arm or an elongated body or loop member configured to be passively secured within the heart. The stabilizing extension is located at the end of the intracardiac device opposite the electrode. The appendage arm includes a distal end upon which an electrode is located. The electrode is a passive electrode that is configured to simply rest against a selected activation site or an active fixation electrode that is configured to be secured to the tissue at the activation site. Further, the stabilizer arm has a distal end that is positioned in the superior vena cava (SVC), wherein the stabilizer arm extends across the whole right atrium. In this position, the stabilizer arm applies a quite high force to the SVC wall leading to an increased risk of injury of the thin tissue of the heart and the vein in this region.

Accordingly, there is the need for implantable intracardiac devices for atrial use having a fixation mechanism with a lower risk of injury and at the same time providing a robust attachment to the heart's tissue and low manufacturing effort and costs.

The above problem is solved by the intracardiac device for implantation within the appendage of an atrium of a patient's heart defined in claim 1.

In particular, the intracardiac device according to the invention comprises a housing and an electrode projecting from the distal end of the housing, wherein a fixation structure is located at the proximal region of the housing and extending therefrom, wherein the fixation structure comprises
- at least two flexible arms, wherein the proximal end of each flexible arm is adapted in its deployed state such that it is in contact and supported by the atrial appendage wall, wherein each flexible arm is preloaded such that the intracardiac device and its electrode is pressed against the atrial appendage wall in the region of a distal tip of the appendage, and/or
- at least one coil spring arm, wherein each coil spring arm is adapted in its deployed state such that at least two sections of each coil spring arm are in contact and supported by the atrial appendage wall, wherein the coil spring arm is preloaded such that the intracardiac device and its electrode is pressed against the atrial appendage wall in the region of a distal tip of the appendage.

The atrial appendage may be the right atrial appendage or the left atrial appendage. The distal tip of the appendage is the back end or rearward end of the appendage.

The proximal region comprises a proximal end of the housing and a proximal section of the outer shell surface of the housing, e.g. the proximal third of the whole length of the outer shell or less or the proximal fourth of the whole length of the outer shell. The proximal end of the housing is the furthest proximal surface or point of the housing, e.g. the proximal end face. The housing may be cylindrically or conically formed.

The inventors noticed that atrial intracardiac leadless pacemaker implantation requires reliable fixation to ensure electrode contact with cardiac tissue. Atrial fixation presents unique challenges compared to ventricular fixation, since the atrial wall is thin and sparsely covered with pectinate muscle formations. The proposed intracardiac device provides a novel atrial fixation for an intracardiac leadless pacemaker using a fixation structure located at the proximal end of the device that interacts with a surrounding atrial wall of an appendage creating back pressure and pushing the device into the atrial appendage so that the electrode at the opposite (distal) end of the device has a good and reliable mechanical and electrical contact with the heart's tissue. At least one coil spring arm or each one of the at least two flexible arms are preloaded and due to this preloading and the support effect of the surrounding atrial wall of the appendage, a back pressure of the surrounding appendage wall is caused which presses the intracardiac device and its electrode against the atrial appendage wall in the region of the distal tip of the appendage.

The proposed invention uses the back pressure of the appendage wall against the preloaded coil spring arm or the at least two preloaded flexible arms caused by the preloading of these arms to ensure reliable electrode contact with the atrial tissue. The fixation structure which protrudes from the housing of the intracardiac device engages with the atrial wall in the respective appendage, pushes the electrode into the distal tip of the appendage due to its preloading, and maintains electrode-tissue contact. The fixation structure extends from the back of the intracardiac device (i.e. proximal region of the device) and attaches within the pectinate muscle network that lines the walls of the appendage.

Using a fixation structure that extends from the proximal region of the device to push the electrode against the distal tip of the respective appendage offers a superior possibility of safe and reliable atrial implantation. The major advantage of applying back pressure to maintain electrode-tissue contact is that it utilizes the atrial anatomy of the respective appendage. The fixation structure that extends from the device and interacts with the appendage wall, positions the intracardiac leadless pacemaker in the pocket of the appendage so that it is supported by the natural shape of the cavity. Furthermore, the shape and positioning of the extensions are designed to get caught in the pectinate muscles that cover the thin atrial walls and create a secure attachment. Leveraging the unique anatomy of the atrial appendage to apply back pressure on the device is an advantageous strategy to provide atrial fixation in an intracardiac device, for example a leadless, intracardiac pacemaker.

Particularly, the fixing structure may be collapsible or foldable form the deployed state to a collapsed state, which may facilitate or enable a vascular delivery of the intracardiac device of the invention, e.g. by means of an implantation catheter. Likewise, the fixing structure may be expandable form the collapsed state into the deployed state, preferably self-expanding. For example, the fixing structure may substantially made of or may comprise a super-elastic material such as nitinol, wherein the fixing structure is in the deployed state when unconstrained and in the collapsed state when constrained, e.g. by a sheath of an implantation catheter. By removing the constraint, e.g. by pulling away the sheath from the fixing structure, the fixing structure may self-expand into the deployed state.

The implantable intracardiac device, for example a leadless pacemaker, may comprise a cylindrical housing and a pin-shaped electrode projecting from the distal end of the housing. Further, a header assembly may be arranged at and attached to the distal end of the housing of the intracardiac device such that the electrode projects through the header assembly, i.e. a respective through-going or complete opening of the header assembly. The opening may be a central opening. The cylindrical housing comprises the electronics module having a processor and an energy source (e.g. a battery or coil (for wireless charging)) and, if applicable, a communication component such as an antenna. The processor may be adapted to process signals determined from the patient's body or received from the surrounding environment and/or to produce signals for treatment of the patient's heart. Such signals may comprise electrical stimulation in the form of pulses in order to generate a physiologically appropriate heartrate, shocks for cardioversion or defibrillation in order to restore a more normal heart rhythm and/or other electrical or electromagnetic signals to the heart or its surrounding tissue. Such signals are transformed and transmitted by the electronic module and may be applied by the pin-shaped electrode to the heart or its surrounding tissue. The pin-shaped electrode is electrically connected to the electronics module and the energy source. The hermetically sealed housing may comprise biocompatible material and/or electrically conducting material, e.g. titanium, and may function as another electrode.

In one embodiment each arm of the at least two flexible arms
a. is a straight arm,
b. is a wire loop,
c. has a different size adapted to the shape of the surrounding appendage wall and has, for example, an S-form or a U-form, or
d. is a flared tine.

The fixation structure may comprise more than two flexible arms, e.g. four arms or six arms, or any combination of above types at least two flexible arms, for example two straight arms and two arms formed as a wire loop. If there is a combination of different types of flexible arms, any two flexible arms of the same type may be located opposite to each other or in a distance of 120 degrees with regard to the circumference of the housing of the intracardiac device.

As indicated above, in one embodiment, each arm of the at least two flexible arms is a straight arm. The pretension or preloading of each arm is provided in the manner of a tension spring clamped at its distal end - the end with which it is attached to the housing of the intracardiac device. The straight arm pivots about its distal end point and/or bends against spring force created by the preload directed outwards, i.e. away from a longitudinal axis of the intracardiac device. Such arm is deployed so that it contacts the atrial appendage wall and pushes the device electrode into the distal tip of the appendage. For example, four arms of equal length and spacing could be used to engage with the pectinate muscle on different surfaces of the right atrial appendage.

As indicated above, in one embodiment, each arm of the at least two flexible arms is a wire loop starting and ending at the device housing. This embodiment uses a wire loop to attach the device to the atrial wall and to maintain electrode-tissue contact. The wire loop extends from the proximal region of the device housing (e.g. its shell surface) so that the loop or eyelet at the proximal end of the loop interacts with the atrial appendage wall, and applies back pressure on the device in the same manner as the straight arm explained above forming a tension spring clamped at the distal end of the loop. This fixation structure allows for several possible points of engagement with the atrial wall providing a better distribution of compression force at the atrial wall due to the larger support area caused by the loop. For example, four loops of equal length and spacing could start at the device body so that both ends of each loop meet at the distal section of the device. The loops may be attached to the proximal end of the housing, as well.

As indicated above, in one embodiment, each arm of the at least two flexible arms has different size adapted to the shape of the surrounding appendage wall and has, for example, an S-form or a U-form. The different sizes of the at least two flexible arms may be in the range of 0.5 cm to 4 cm. These arms would allow engagement with the unique pectinate muscle pattern at the respective appendage wall. For example, six flexible arms could be used to position the device electrode in the distal tip of the appendage with some of the arms caught in the pectinate muscle near the back end of the device and others engaged further away from the device near the opening of the appendage. As indicated above, these arms may act as tension springs clamped at their distal ends or, alternatively or additionally, provide additional flexibility from their curved form, for example the S-form and the U-form.

As indicated above, in one embodiment, each arm of the at least two flexible arms is a flared tine. This embodiment uses tine-shaped extensions that open away from the distal tip of the respective appendage or the distal end of the intracardiac device. The tines are flared to make contact with the atrial wall at each surface at the proximal end of each tine. The proximal end of each tine tangentially approaches the wall of the atrial appendage so that there is an advantageous distribution of forces in the contact region and the risk of injury is minimized. In contrast to ventricular tines that attach to the tissue near the device electrode and pull the device to the tissue, the tines in this design instead engage with the atrial wall proximal to the electrode and push the device against the tissue. For example, there could be four tines of equal size and spacing used to attach to the appendage wall. The tines may have a length in the range of 1 cm to 4 cm.

As indicated above, the fixation structure may comprise at least one coil spring arm, wherein each coil spring arm is adapted in its deployed state such that at least two sections of each coil spring arm are in contact and supported by the atrial appendage wall and are preloaded such that the back pressure of the atrial appendage wall provided to the device presses the electrode against the atrial appendage wall. The spring extends from the back of the device, i.e. its proximal end. It may form a uniform diameter or a gradually increasing diameter with each turn to match the increasing size of the appendage (i.e. its inner diameter into a direction perpendicular to the longitudinal axis of the housing or the intracardiac device) away from the distal tip with the sensing/pacing electrode. The coil spring may comprise at least 1 and 1/2 turns, preferably at least 2 turns. This example provides several spots for wall engagement with every turn of the coil. For example, the spring could use two whole turns starting from the back end of the device to fasten to the pectinate muscle along the appendage wall. As indicated above, in one embodiment, the at least one coil spring arm is a conical spring.

All embodiments would also be paired with an implantation catheter that delivers the device and allows for positioning of the anchoring structures so that adequate pressure is applied on the electrode-tissue interface.

In one embodiment, the furthest proximal end of each flexible arm or each coil spring arm takes a blunt form such as, for example, a rounded kink or a ball form in order to avoid any injury of the appendage wall.

In one embodiment, the diameter of each of the at least two flexible arms is in the range of 0.5 cm to 4 cm.

In one embodiment, the diameter of each of the at least one coil spring arm is in the range of 0.5 cm to 4 cm.

In one embodiment, the material of the at least two flexible arms or the at least one coil spring arm comprises or is substantially made of a super-elastic material and/or shape memory alloy, particularly nitinol.

In one embodiment, the at least two flexible arms or the at least one coil spring arm is welded to the proximal region of the housing, for example at the proximal end face of the housing and/or the outer shell surface of the housing. In an alternative embodiment, the at least two flexible arms or the at least one coil spring arm is attached via lock in place fittings with the housing.

The present invention will now be described in further detail with reference to the accompanying schematic drawing, wherein
Fig. 1 shows a first embodiment of the intracardiac device of the invention in a deployed state in a side view within the right atrial appendage,
Fig. 2 shows a second embodiment of the intracardiac device of the invention in a deployed state in a side view within the right atrial appendage,
Fig. 3 shows a third embodiment of the intracardiac device of the invention in a deployed state in a side view within the right atrial appendage,
Fig. 4 shows a fourth embodiment of the intracardiac device of the invention in a deployed state in a side view within the right atrial appendage, and
Fig. 5 shows a fifth embodiment of the intracardiac device of the invention in a deployed state in a side view within the right atrial appendage.

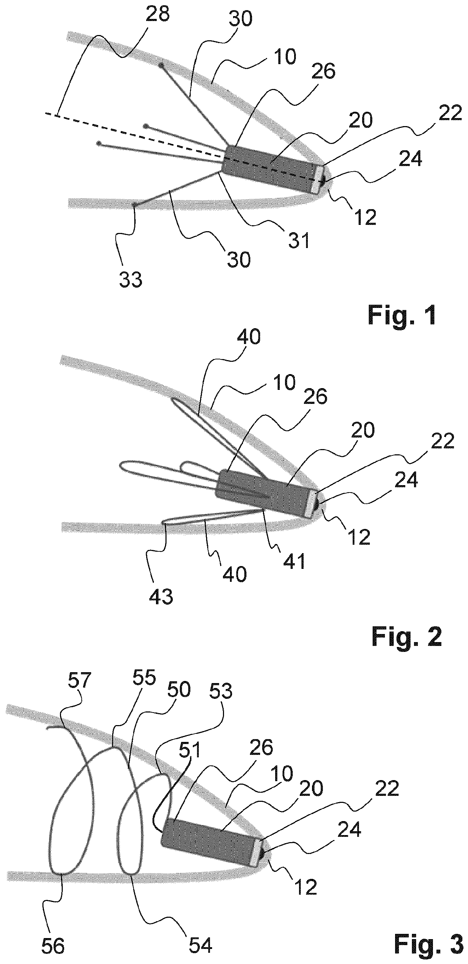

Fig. 1 illustrates a first embodiment of an implantable intracardiac device according to the invention, e.g. a leadless pacemaker in a deployed position within the right atrial appendage 10 of a patient. The intracardiac device comprises a cylindrical housing 20. A header 22 and a pin-shaped electrode 24 are located at the distal end of the housing 20. The pin-shaped electrode protrudes through the header 22 and from its distal end face.

The housing 20 of the intracardiac device contains a battery and an electronic module comprising a processor and ensures hermetic sealing of these components. These components are electrically connected to the electrode 24 and provide the electrical stimulation of the heart or processing of electrical signals determined from the heart. Further, the housing 20 may contain components for communication such as an antenna.

There is a fixation structure at the proximal region 26 of the housing 20 comprising four straight arms 30 protruding from the proximal end face of the housing 20. Each straight arm 30 is attached with its distal end 31 to the proximal end face of the housing 20. Each straight arm 30 can be pivoted about the attachment point and/or bended against a spring force caused by a preload directed outwards, i.e. away from the longitudinal axis 28 of the intracardiac device or its housing. The proximal end 33 of each straight arm 30 comprises a blunt form shaped as a ball which contacts the atrial appendage wall and is clamped in the atrial appendage 10. The back pressure of the wall of the right atrial appendage 10 caused by the preload of the four straight arms 30 and the support of the appendage wall pushes the intracardiac device into a distal tip 12 of the right atrial appendage 10 thereby providing reliable mechanical and electrical contact of the electrode 24 to the tissue of the patient's heart.

The embodiment shown in Fig. 2 has a different fixation structure in the form of four wire loops 40. Each wire loop 40 starts and ends at its distal end 41 and forms a loop or eyelet at its proximal end 43. With this loop or eyelet the intracardiac device is in contact with the wall of the atrial appendage 10 and provides a fixation and a back pressure to the intracardiac device in order to form a reliable mechanical and electrical connection of the electrode 24 to the wall in the region of the distal tip 12 of the atrial appendage 10.

In Fig. 3 another fixation structure is used in order to create the necessary back pressure to the intracardiac device. The fixation structure comprises one coil spring arm 50 attached with its distal end 51 to the proximal end face of the housing 20 of the intracardiac device 20. The coil spring arm 50 forms little more than two turns, wherein the diameter gradually increases to match the increasing inner diameter into the proximal direction. At least in the regions 53, 54, 55, 56, 57 the coil spring arm 50 contacts the atrial appendage wall and provides a preload against it.

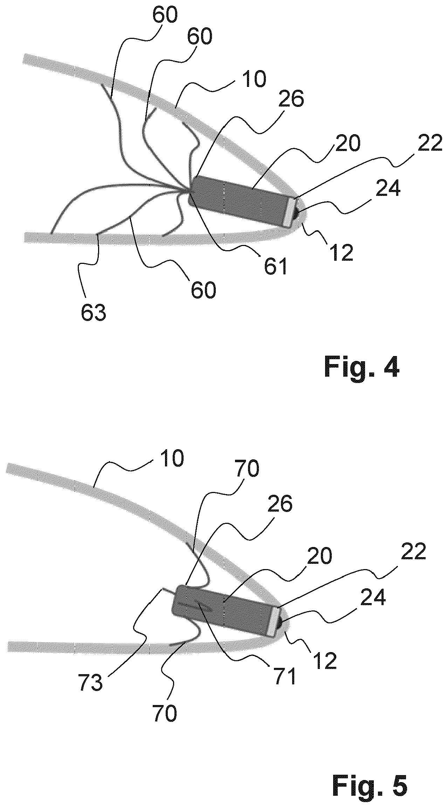

The fixation structure of the embodiment shown in Fig. 4 comprises six flexible arms 60 with different sizes, for example dimensions in the range of 0.5 cm to 4 cm. Each flexible arm 60 is attached with its distal end 61 to the proximal end surface of the housing 20 of the intracardiac device. Further, the flexible arms 60 have a U-form or an S-form causing the necessary flexibility to provide the spring force caused by the preload in order to press the electrode 24 against the distal tip 12 of the atrial appendage 10. Each flexible arm 60 is in contact with its proximal end 63with the wall of the atrial appendage 10 and in engagement with the unique pectinate muscle pattern at the respective appendage wall.

Finally, in Fig. 5 another embodiment of an intracardiac device is depicted. This embodiment comprises a fixation structure with four flared tines 70 attached with its distal end 71 to the proximal region of the housing 20, in particular its outer shell. The tines 70 are flared, each having a U-shape which opens into proximal direction - i.e. away from the distal direction. The proximal end 73 of each tine 70 is engaged with the wall of the atrial appendage 10 proximal from the electrode. Further, the proximal end 73 of each tine 70 tangentially approaches the wall of the atrial appendage 10. The spring force provided by each tine 70 acts radially outwardly, into the direction of the atrial appendage wall and thereby pushes the intracardiac device into the direction of the distal tip of the respective atrial appendage (i.e. the right atrial appendage).

The above fixation structures preferably comprise or essentially consist of a biocompatible, particularly super-elastic material, such as nitinol.

The above explained fixation structures may be welded to the outer surface of the housing 20 of the intracardiac device. Other attachment mechanisms may comprise lock in place fittings with the device housing.

In the above embodiments and the respective figures the same reference numbers refer to the same elements which are explained with regard to Fig. 1 in detail. It is referred to this explanation.

The above embodiments of intracardiac devices are capable of delivering paced stimulation and sensing intrinsic electrical activity from a unit entirely contained in the right atrium. Particularly, the fixation structure protruding or extending from the proximal region of the device housing, engages with the atrial wall such a back pressure from the atrial wall is applied on the intracardiac device via the fixation structure, and thereby the device electrode is pushed against the distal tip of the appendage. The above embodiments further provide a simple and cost effective solution of the above problem.

## Claims

1. An intracardiac device for implantation within the appendage (10) of an atrium of a patient's heart comprising a housing (20) and an electrode (24) projecting from the distal end of the housing (20), wherein a fixation structure is located at the proximal region of the housing and extending therefrom, wherein the fixation structure comprises
- at least two flexible arms (30, 40, 60, 70), wherein the proximal end (33, 43, 63, 73) of each flexible arm is adapted in its deployed state such that it is in contact and supported by the atrial appendage wall, wherein each flexible arm is preloaded such that the intracardiac device and its electrode (24) is pressed against the atrial appendage wall in the region of a distal tip (12) of the appendage, and/or
- at least one coil spring arm (50), wherein each coil spring arm is adapted in its deployed state such that at least two sections (53, 54, 55, 56, 57) of each coil spring arm are in contact and supported by the atrial appendage wall, wherein the coil spring arm is preloaded such that the intracardiac device and its electrode (24) is pressed against the atrial appendage wall in the region of a distal tip (12) of the appendage.

2. The intracardiac device according to claim 1, wherein each arm of the at least two flexible arms
a. is a straight arm (30),
b. is a wire loop (40),
c. has a different size adapted to the shape of the surrounding appendage wall and has, for example, an S-form or a U-form, or
d. is a flared tine (70).

3. The intracardiac device according to any of the previous claims, wherein the furthest proximal end (33, 43, 63, 73) of each flexible arm or each coil spring arm take a blunt form such as, for example, a rounded kink or a ball form.

4. The intracardiac device according to any of the claims 1 and 3, wherein the at least one coil spring arm (50) is a conical spring.

5. The intracardiac device according to any of the previous claims, wherein the length of each of the at least two flexible arms (30, 40, 60, 70) is in the range of 0.5 cm to 4 cm.

6. The intracardiac device according to any of the previous claims, wherein the diameter of each of the at least one coil spring arm (50) is in the range of 0.5 cm to 4 cm.

7. The intracardiac device according to any of the previous claims, wherein the material of the at least two flexible arms (30, 40, 60, 70) or the at least one coil spring arm (50) comprises or is substantially made of a super-elastic material and/or a shape memory alloy, such as nitinol.

8. The intracardiac device according to any of the previous claims, wherein the at least two flexible arms (30, 40, 60, 70) or the at least one coil spring arm (50) are/is welded to the housing (20), for example at the proximal end face of the housing (20) and/or the outer shell surface of the housing (20), or attached via lock in place fittings with the housing (20).
